# EUROPEAN PATENT SPECIFICATION

(11) **EP 4 111 984 B1**
(45) Date of publication and mention of the grant of the patent: **18.12.2024**
(21) Application number: 21767819.2
(22) Date of filing: 09.03.2021
(51) Int. Cl.: A61B 10/00, A61B 5/11, A61B 5/00

(54) **INFORMATION PROCESSING METHOD, COMPUTER PROGRAM, INFORMATION PROCESSING DEVICE, AND INFORMATION PROCESSING SYSTEM**
INFORMATIONSVERARBEITUNGSVERFAHREN, COMPUTERPROGRAMM, INFORMATIONSVERARBEITUNGSVORRICHTUNG UND INFORMATIONSVERARBEITUNGSSYSTEM
PROCÉDÉ DE TRAITEMENT D'INFORMATIONS, PROGRAMME INFORMATIQUE, DISPOSITIF DE TRAITEMENT D'INFORMATIONS, ET SYSTÈME DE TRAITEMENT D'INFORMATIONS

(30) Priority: 10.03.2020 JP 2020041076
(43) Date of publication of application: 04.01.2023
(73) Proprietor: Terumo Kabushiki Kaisha, Tokyo 151-0072 (JP)
(72) Inventor: HONMA, Yasuyuki, Ashigarakami-gun, Kanagawa 259-0151 (JP); MAEDA, Naoyuki, Ashigarakami-gun, Kanagawa 259-0151 (JP)
(74) Representative: Casalonga
(86) International application number: PCT/JP2021/009229
(87) International publication number: WO 2021/182455

(56) References cited:
- WO-A1-2019/200393
- AU-A1- 2017 305 321
- GB-A- 2 285 135
- JP-A- 2005 348 893
- JP-A- 2018 171 443
- JP-A- 2019 535 069
- KR-A- 20150 032 956
- US-A1- 2015 018 723
- US-A1- 2015 164 377
- US-A1- 2019 029 606

## Description

### Technical Field

The present technology relates to an information processing device, a computer program and an information processing system.

### Background Art

US 2015/164377 A1 relates to a device comprising a processor, an output device, and at least one accelerometer, angle, location, direction, or physiological state sensor.

AU 2017305321 A1 relates to methods and kits for detecting, screening, quantifying or localizing the etiology for reduced or impaired cranial nerve function or conduction; localizing a central nervous system lesion; detecting, diagnosing or screening for increased intracranial pressure, pressure or disruption of central nervous system physiology as seen with concussion; or detecting, diagnosing, monitoring progression of or screening for a disease or condition featuring increased intracranial pressure or concussion by tracking eye movement of the subject.

US 2015/018723 A1 relates to an apparatus for early detection of paralysis based on motion sensing including a sensing part and a motion disorder determination unit US 2019/029606 A1 relates to a real-time automated method to diagnose and/or detect stroke including the steps of continuously measuring natural limb activity, conveying the measurements to a cloud based real-time data processing system, identifying patient specific alert conditions, and determining solutions for acting upon needs of the patient.

WO 2019/200393 A1 relates to real-time head and spine alignment, position and other physiological parameters that are detected to measure human being health and wellness status. The system includes wearable universal sensor modules that are attached and detached easily from receiver units having different formats for different body locations depending on their physiological functions.

The cerebral infarction is a state in which a cerebral blood vessel is blocked or stenosed to cause cerebral ischemia, and brain tissue is in a necrotic state. The cause of the cerebral infarction is mainly divided into thrombotic, embolic, and hemodynamic properties, and is specifically divided into atherothrombotic cerebral infarction, cardiogenic cerebral infarction, lacunar infarction, and others. In a case where cerebral infarction has occurred, cells downstream of the blocked or stenosed cerebral blood vessel are necrotic, so that symptoms such as paralysis, disturbance of speech, blindness, dizziness, incontinence, consciousness disorders, and the like are generated.

The cerebral infarction is enlarged as time passes, and thus becomes more serious as the detection is further delayed. Therefore, it is important to detect cerebral infarction at an early stage after these symptoms have occurred. PTL 1 describes a method of detecting cerebral infarction by measuring the concentration of nick β2 glycoprotein I in a body fluid sample.

### Citation List

### Patent Literature

PTL 1: JP-A-2003-121444

### Summary of Invention

### Technical Problem

A subject actively uses a diagnosis using the method as in PTL 1 to allow cerebral infarction to be detected. Meanwhile, symptoms indicating the occurrence of cerebral infarction or cerebral ischemia suddenly appear in daily life in many cases. Moreover, a symptom such as transient cerebral ischemic attack is transient and disappears in a short time. In such a case, even if a symptom of cerebral infarction or cerebral ischemia appears, the subject has no subjective symptom or overlooks the symptom, and a case where the cerebral infarction is not detected can occur.

An object of the present disclosure is to provide an information processing device capable of determining the presence or absence of an abnormal state in which there is a possibility of cerebral infarction in a subject.

### Solution to Problem

In order to solve the above problem, the present invention provides an information processing device according to independent claim 1. The dependent claims relate to advantageous embodiments.

### Advantageous Effect of Invention

With the present disclosure, it is possible to determine the presence or absence of an abnormal state in which there is a possibility of cerebral infarction in a subject.

### Brief Description of Drawings

[Fig. 1] Fig. 1 is a schematic diagram of an a information processing system in a first embodiment.
[Fig. 2] Fig. 2 is a block diagram illustrating a configuration of an information processing device.
[Fig. 3A] Fig. 3A is an explanation diagram illustrating an example of a record layout of a subject information DB.
[Fig. 3B] Fig. 3B is an explanation diagram illustrating an example of a record layout of a motion information DB.
[Fig. 3C] Fig. 3C is an explanation diagram illustrating an example of a record layout of a reference motion information DB.
[Fig. 4] Fig. 4 is a block diagram illustrating a configuration of a detection device.
[Fig. 5] Fig. 5 is a block diagram illustrating a configuration of a terminal device.
[Fig. 6] Fig. 6 is a flowchart illustrating one example of a processing procedure of reference motion information acquisition.
[Fig. 7] Fig. 7 is a flowchart illustrating one example of a determination processing procedure of an abnormal state.
[Fig. 8] Fig. 8 illustrates explanation diagrams related to determination processing of an abnormal state.
[Fig. 9] Fig. 9 is a view illustrating one example of a notification screen that is displayed in a display unit.
[Fig. 10] Fig. 10 is a flowchart illustrating one example of a processing procedure relating to warning information.
[Fig. 11] Fig. 11 is a view illustrating one example of a warning screen that is displayed in the display unit.
[Fig. 12] Fig. 12 is a block diagram illustrating a configuration of a detection device in a second embodiment.
[Fig. 13] Fig. 13 is a flowchart illustrating one example of a determination processing procedure of an abnormal state in the second embodiment.

### Description of Embodiments

Embodiments of the present invention are specifically described with reference to the drawings.

### (First Embodiment)

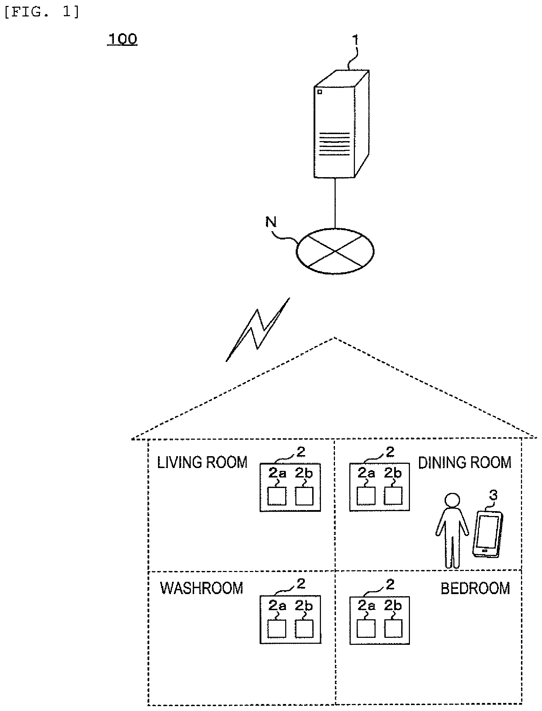

Fig. 1 is schematic diagram of an information processing system 100 in a first embodiment. The information processing system 100 in the present embodiment is a system that determines a possibility of cerebral infarction from motion information on a subject. The information processing system 100 includes an information processing device 1, and a motion detection sensor 2a that is provided to a detection device 2. The information processing device 1 and the detection device 2 can transmit and receive information via a network N such as the Internet. The information processing device 1 further communication-connected with a terminal device 3 via the network N.

The information processing device 1 determines, based on motion information on a subject acquired from the detection device 2, a risk of a symptom, a sign, or the like of cerebral infarction or cerebral ischemia in the subject, in other words, a possibility of cerebral infarction. The information processing device 1 provides information in accordance with a determination result via the terminal device 3 and the like.

The motion information is a parameter representing a motion amount of the subject. The motion amount is, for example, a movement amount (movement speed) per unit time, a movement distance, a shake, an angular speed, and the like of each site such as limbs and a head of the subject, and can be obtained from an image in which the subject is photographed as will be described later. Note that, as will be described later, the motion amount is not limited to the one obtained from an image, but may be data detected by a microwave sensor, a millimeter wave sensor, an ultrasound sensor, an acceleration sensor, or the like.

The detection device 2 is provided in a house of a subject, for example, and detects a motion of the subject in a daily life. The detection device 2 is provided with the motion detection sensor (motion detection device) 2a, and transmits motion information detected by the motion detection sensor 2a to the information processing device 1. A plurality of the motion detection sensor 2a are preferably provided in a plurality of areas such as a living room, a dining room, a kitchen, a washroom, and a bedroom inside the house of the subject, for example. The motion detection sensors 2a may be disposed at positions where the subject can live without being conscious of the sensors. The detection device 2 may be provided with, in addition to the motion detection sensor 2a, a living body information detection sensor (living body information detection device) 2b. The living body information detection sensor 2b will be described in details in another embodiment.

The motion detection sensor 2a is, for example, a camera that photographs an image within an imaging target region. The motion detection sensor 2a includes an imaging element such as an charge coupled device (CCD) image sensor, a lens, or the like, and acquires motion information that is image data by photoelectrically converting light entered through the lens by the imaging element. The motion detection sensor 2a may be a stereo camera including a plurality of imaging elements. By analyzing image data photographed by the motion detection sensor 2a, it is possible to two-dimensionally or three-dimensionally recognize a motion of a subject included in the image data. The motion detection sensor 2a is not limited to the one that uses the abovementioned camera, but may detect a position, a shape, and the like of the subject by using an ultrasound sensor, a microwave sensor, a millimeter wave sensor, or a laser radar sensor, for example. The motion detection sensor 2a may measure an eye movement of the subject by using a line-of-sight detection sensor, and detect a line-of-sight direction, a view point, and the like. The detection device 2 acquires a detection value of the motion detection sensor 2a.

Although the configuration in which the detection device 2 is disposed in the house of the subject has been described in the above, the present embodiment is not limited thereto. For example, the detection device 2 may be configured as a wearable device, and may be mounted to the subject. The detection device 2 has a shape of a spectacle type, for example. The detection device 2 is provided with a motion sensor serving as the motion detection sensor 2a that measures the acceleration, the angular speed, and the geomagnetism respectively with three axes, and detects a motion of the subject. The detection device 2 may be provided with a myoelectric potential sensor serving as the motion detection sensor 2a at a position where the myoelectric potential sensor is in contact with the nose, the head, or the like of the subject in a mounted state, and may detect a face myoelectric potential of the subject. The detection device 2 may be provided with a myoelectric potential sensor serving as the motion detection sensor 2a at a position where the myoelectric potential sensor is in contact with the nose of the subject in a mounted state, for example, and may detect a line-of-sight direction and a motion such as a blink by detecting an eye potential of the subject. The detection device 2 is not limited to the one having a shape of a spectacle type, for example, but may have a band shape and be mounted to an arm, a leg, a waist, or the like of the subject. The detection device 2 may have a sheet shape with the elasticity, for example, and may be stuck to the body of the subject. Moreover, the detection device 2 may be configured as a cavity device of a contact lens type, a mouthpiece type, or the like, for example, and may be mounted to the body cavity of the subject.

A plurality of the detection devices 2 may be mounted to the subject. The detection device 2 may include a combination of several types of the motion detection sensors 2a, and may acquire detection values by the several types of the sensors. For example, the detection device 2 may detect information on the whole imaging region including a subject by a camera, and may detect a motion of the subject specified based on the detected imaging data by a laser radar sensor and the like.

In the information processing system 100, the motion detection sensor 2a detects motion information specially related to a motion that is repeatedly performed by the subject on a daily basis. The detected motion information is analyzed, and a motion state in the left half body and a motion state in the right half body of the subject are acquired and compared with motion states in the left half body and the right half body of the subject in normal times, thereby determining a possibility of cerebral infarction in the subject.

For example, specially, the appearance of a symptom such as sudden numbness of the face or the limb, poor eyesight, or slurred speech at one side of the body is highly probably caused because a part of the brain does not function. Accordingly, by detecting an occurrence of a sudden left-right difference in the motion of the subject, it is possible to identify a possibility of an onset or a sign of cerebral infarction or cerebral ischemia. The configuration and detailed processing content of such the information processing system 100 will be described below.

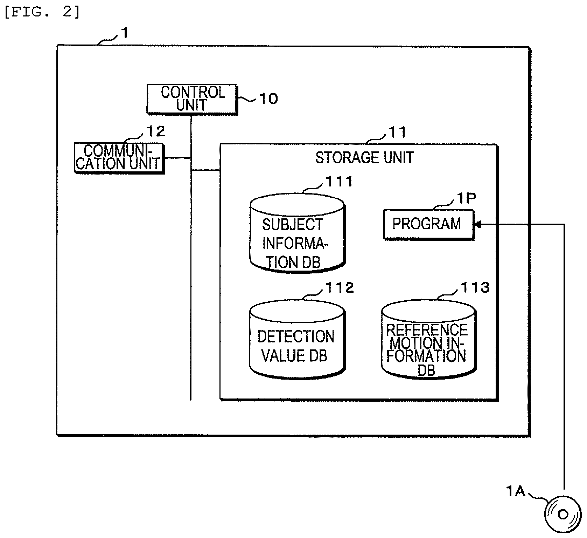

Fig. 2 is a block diagram illustrating a configuration of the information processing device 1. A server computer is used as the information processing device 1. The information processing device 1 is provided with a control unit 10, a storage unit 11, and a communication unit 12. The information processing device 1 is described as one server computer for easy explanation, however, the function or processing thereof may be distributed by a plurality of server computers, or the information processing device 1 may be one of a plurality of server computers (instances) that are virtually generated in one large computer.

The control unit 10 is a processor in which one or a plurality of central processing units (CPUs), graphics processing units (CPUs), and the like are used, and controls respective constituent units by using an embedded memory such as a read only memory (ROM) or a random access memory (RAM) to execute processing.

The communication unit 12 is an communication interface that implements communication via the network N. The control unit 10 can transmit and receive information to and from the detection device 2, other external devices, and the like via the network N, by the communication unit 12.

The storage unit 11 includes, for example, a nonvolatile memory such as a hard disk or a solid state drive (SSD). In the storage unit 11, programs including a program 1P and data to which the control unit 10 refers are stored. The control unit 10 reads and executes the program 1P to cause a general-purpose server computer to function as an information processing device specific to the present disclosure. The program 1P to be stored in the storage unit 11 may be a form in which the program 1P is recorded on a recording medium in a computer-readable manner. The storage unit 11 stores therein the program 1P read from a recording medium 1A by a reading device, which is not illustrated. Moreover, the program 1P may be downloaded from an external computer, which is not illustrated, connected to a communication network, which is not illustrated, and may be stored in the storage unit 11. Note that, the storage unit 11 may include a plurality of storage devices, or may be an external storage device connected to the information processing device 1.

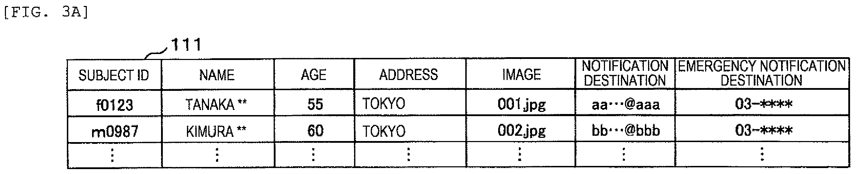

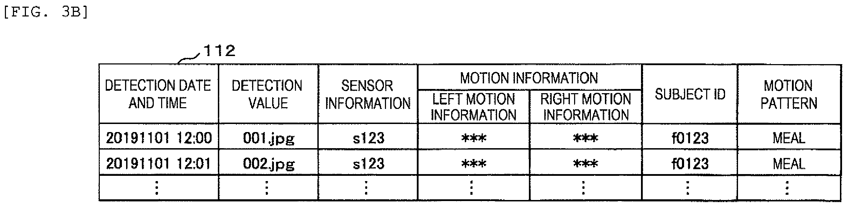

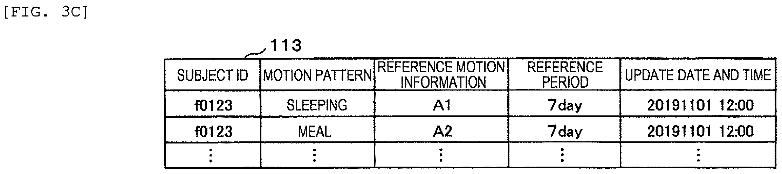

Moreover, in the storage unit 11, a subject information data base (DB) 111, a detection value DB 112, a reference motion information DB 113, and the like are stored. Fig. 3A is an explanation diagram illustrating an example of a record layout of the subject information DB 111, Fig. 3B is an explanation diagram illustrating an example of a record layout of the detection value DB 112, and Fig. 3C is an explanation diagram illustrating an example of a record layout of the reference motion information DB 113.

The subject information DB 111 is a data base that stores therein information on subjects who use the information processing system 100. For example, in the subject information DB 111, as illustrated in Fig. 3A, information such as a subject ID of each of a plurality of subjects, a name, an age, an address of the subject, image data in which a face of the subject is photographed, a notification destination, and an emergency notification destination is stored in association with one another. The notification destination includes a notification destination to which a notification is output in a case where an abnormal state having a possibility of cerebral infarction in the subject has occurred. In the notification destination, for example, a MAC address of the terminal device 3 owned by the subject, a mail address, and the like are stored. The emergency contact address includes a notification destination in an emergency at which a possibility of cerebral infarction in the subject is high. In the emergency contact address, for example, a telephone number or the like of a person other than the subject is stored.

The detection value DB 112 is a data base in which a detection value, motion information, and the like detected by the motion detection sensor 2a are stored. As illustrated in Fig. 3B, in the detection value DB 112, for example, information such as detection date and time, sensor information, a detection value, motion information, a subject ID, and a motion pattern is stored in association with one another in a time-series manner. Each motion information to be stored in the detection value DB 112 is acquired and stored by the control unit 10 for every time when a control unit 20 of the detection device 2 acquires motion information, and transmits the acquired motion information to the information processing device 1.

In the sensor information, identification information for identify the motion detection sensor 2a that has output a detection value is included, and a unique ID of the sensor is stored, for example. In the detection value, a detection value by the motion detection sensor 2a is stored.

The column of the motion information includes a column of left motion information and a column of right motion information. In the column of the left motion information and the column of the right motion information, motion information indicating a motion at the left half body and motion information indicating a motion at the right half body in the subject are respectively stored. Each of the left motion information and the right motion information is a parameter indicating a motion amount in a corresponding site of the subject, is data such as a movement speed, a movement distance, a shake, an angular speed, and the like of the corresponding site, as mentioned above. The corresponding site is a site of a human body such as a joint that serves as a feature point for specifying a posture of the subject. In the present embodiment, as one example, movement amounts (movement speeds) of a left hand and a right hand of the subject per unit time are respectively stored in the column of the left motion information and the column of the right motion information.

In the subject ID, identification information for identifying a subject of the motion information is included, and a subject ID is stored, for example. The subject ID is specified such that a face feature amount of a photographed subject is extracted from an image photographed for detecting motion information, and is subjected to pattern matching with a pre-registered face feature amount of the subject. The subject ID may be specified by using another method including machine learning.

The motion pattern is a type of a motion that can be daily performed by the subject, and can include, for example, sleeping, meal, toothbrushing, television viewing, cooking, and walking. The motion pattern is specified, for example, by performing pattern matching of a position, a posture, or a motion amount of the subject inside a room. The motion pattern may be specified by using another method including machine learning.

The reference motion information DB 113 is a data base in which reference motion information indicating a motion of a subject in a normal state is stored. The information processing device 1 acquires, when determining a possibility of cerebral infarction in a subject, reference motion information obtained by collecting motion information on the subject in a predetermined period, and stores the reference motion information in the reference motion information DB 113. The information processing device 1 determines the presence or absence of an abnormal state by comparing the reference motion information with motion information to be acquired in real time. In the reference motion information DB 113, for example, as illustrated in Fig. 3C, information such as a subject ID, a motion pattern, reference motion information, a reference period, and update date and time is stored in association with one another.

In the motion pattern, information for identifying a motion pattern is included, and names indicating motion patterns such as sleeping, meal, toothbrushing, television viewing, cooking, and walking are stored, for example.

The reference motion information includes motion information on the subject in a reference state obtained by analyzing the motion information in a predetermined period. The reference motion information includes left reference motion information indicating a motion in the left half body and right reference motion information indicating a motion in the right half body. Each of the left reference motion information and the right reference motion information is acquired, for example, in a predetermined period, by collecting motion information including left motion information and right motion information with respect to the identical motion pattern of the subject, and calculating a range and a mean value thereof. The reference motion information may include a plurality pieces of motion information in which information related to time is associated.

The reference period includes a period during which motion information for deriving reference motion information is collected. The reference period is, for example, seven days. The update date and time include the latest information on the date and time when reference motion information has been updated. In the present embodiment, the reference motion information is updated whenever needed based on the motion information that is detected in real time. The use of the reference motion information to be generated based on a most recent motion state of the subject allows the determination in accordance with a state of an individual subject including a temporary injury and a change due to aging, for example. Note that, the storage contents of the subject information DB 111, the detection value DB 112, and the reference motion information DB 113 are not limited to the examples illustrated in Figs. 3A to 3C.

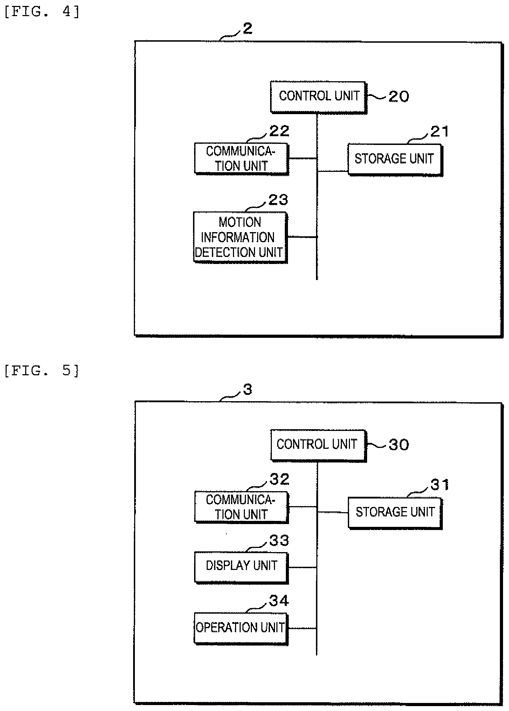

Fig. 4 is a block diagram illustrating a configuration of the detection device 2. The detection device 2 is provided with the control unit 20, a storage unit 21, a communication unit 22, and a motion information detection unit 23.

The control unit 20 is a processor in which one or a plurality of CPUs, GPUs, and the like are used, and controls respective constituent units by using an embedded memory such a ROM or a RAM to execute processing. The storage unit 21 includes, for example, a nonvolatile memory such as a flash memory. In the storage unit 21, information to which the control unit 20 refers is stored. The communication unit 22 is a communication interface that implements communication with the information processing device 1 via the network N.

The motion information detection unit 23 acquires a detection signal by using the motion detection sensor 2a. The motion information detection unit 23 may include an A/D conversion function, and may output a measurement value obtained from the motion detection sensor 2a to the control unit 20.

Fig. 5 is a block diagram illustrating a configuration of the terminal device 3. As the terminal device 3, for example, a smartphone, a tablet terminal, or the like is used. The terminal device 3 may be an information terminal device such as a personal computer. The terminal device 3 is provided with a control unit 30, a storage unit 31, a communication unit 32, a display unit 33, and an operation unit 34.

The control unit 30 includes a processor such as a CPU or a GPU, a memory, and the like. The control unit 30 may be configured as one hardware (SoC: System On a Chip) in which the processor, the memory, the storage unit 31, and the communication unit 32 are integrated. The control unit 30 controls the respective constituent units based on a program stored in the storage unit 31 to execute processing.

The storage unit 31 includes, for example, a nonvolatile memory such as a flash memory. The storage unit 31 stores therein a program and data to which the control unit 30 refers. The communication unit 32 is a communication interface that implements communication with the information processing device 1 via the network N.

The display unit 33 includes a display device such as a liquid crystal panel or an organic electro luminescence (EL) display. The display unit 33 displays various kinds of information in accordance with an instruction from the control unit 30. The operation unit 34 is an interface that receives an operation by a user, and includes, for example, a physical button, a mouse, a touch panel device that is embedded in the display, a speaker, a microphone, and the like. The operation unit 34 receives an operation input from the user, and sends out a control signal in accordance with the operation content to the control unit 30.

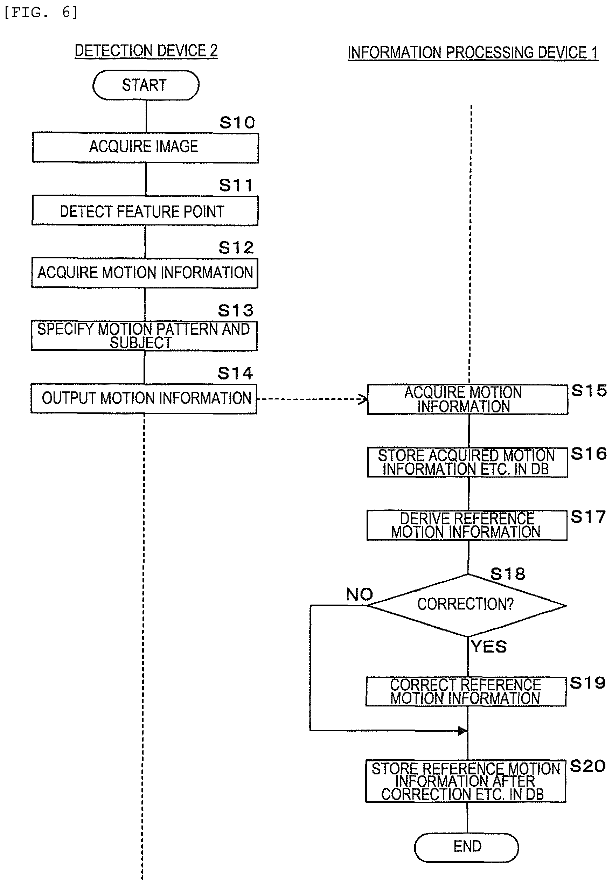

Fig. 6 is a flowchart illustrating one example of a processing procedure of reference motion information acquisition. The processing in Fig. 6 is executed by the control unit 10 of the information processing device 1 and the control unit 20 of the detection device 2. The execution timing of the processing is, for example, timing at which the motion detection sensor 2a detects a signal.

The control unit 20 of the detection device 2 acquires an image in which a subject is photographed from the motion detection sensor 2a in real time or at a predetermined detection interval (for example, for every minute) (Step S10). The control unit 20 detects a feature point corresponding to a corresponding site of the subject from the acquired image (Step S11). For example, the control unit 20 detects a right hand and a left hand of the subject as feature points.

The control unit 20 acquires motion information including right motion information and left motion information based on a detection result of feature points in each image acquired on a time-series basis (Step S12). Specifically, the control unit 20 compares image data acquired on a time-series basis to calculate motion amounts of the corresponding site in the right half body and the left half body included in the images at the previous time and the current time. For example, the detection device 2 respectively compares coordinates of the left hand and the right hand in the previous frame with coordinates of the left hand and the right hand in the current frame, and acquires movement speeds calculated from the movement distance and the detection time, as motion information.

Moreover, the control unit 20 specifies a motion pattern of the subject, and the subject (Step S13). For example, the control unit 20 detects respective sites such as limbs and a head as feature points at Step S11, and from coordinate values of the detected respective feature points, performs pattern matching of a position, a posture, and a motion (gesture) of the subject inside a room, thereby specifying a motion pattern. Moreover, the control unit 20 stores (registers) in advance a subject ID and a face feature amount of the subject in association with each other, extracts a face feature amount at Step S10, and performs pattern matching with the stored face feature amount, thereby specifying the subject ID. The control unit 20 outputs motion information in association with the specified motion pattern, the subject ID, the detection date and time, the sensor ID, the detection value, and the like, to the information processing device 1 (Step S14).

The control unit 10 of the information processing device 1 acquires motion information by the communication unit 12 (Step S15). The control unit 10 stores the acquired motion information in association with the subject ID, the motion pattern, and the like, in the detection value DB 112 (Step S16).

The control unit 10 derives reference motion information indicating a motion of the subject in normal times, based on the collected motion information (Step S17). Specifically, the control unit 10 refers to the detection value DB 112, and acquires motion information on the same subject detected within a predetermined period (for example, seven days) and stored in the detection value DB 112. In this case, the control unit 10 derives reference motion information for each motion pattern, and thus may extract motion information including the identical motion pattern. For example, in a case where basic motion information relative to a motion pattern "meal" is derived, the control unit 10 extracts detection date and time including the most recent one week from the derivation time point, an identical subject ID, and motion information including "meal" as the identical motion pattern. The control unit 10 calculates, based on left motion information and right motion information in the extracted motion information, mean values of the left motion information and the right motion information, and thus derives reference motion information including left reference motion information indicating a reference motion of the left half body, right reference motion information indicating a reference motion of the right half body.

In addition, the control unit 10 compares the derived reference motion information with reference motion information already recorded in advance, and determines whether the reference motion information is to be corrected (Step S18). Specifically, the control unit 10 determines a magnitude relationship of a difference value between the derived reference motion information and the already-recorded reference motion information, and a threshold value set in advance, and determines whether the difference value is equal to or more than the threshold value. If the control unit 10 has determined that the reference motion information is not corrected because the difference value is less than the threshold value (Step S18: NO), the control unit 10 skips the correction processing of the reference motion information.

If the control unit 10 has determined that the reference motion information is corrected because the difference value is equal to or more than the threshold value (Step S18: YES), the control unit 10 executes correction of changing the already-recorded reference motion information to the newly derived reference motion information (Step S19). The control unit 10 may correct the value of the already-recorded reference state information based on a predetermined rule. The control unit 10 stores the reference motion information after the correction and information such as the update date and time, in the reference motion information DB 113 (Step S20), and ends the series of the processing.

In the abovementioned processing, the control unit 10 may execute the processing at Step S17 and the subsequent processing if the feature amount indicating the motion pattern that is included in the acquired motion information is equal to or more than a predetermined value, from the analyzed result at Step S15. In other words, in a case where the acquired motion information corresponds to the motion pattern set in advance, the control unit 10 may execute the derivation processing of the reference motion information.

Note that, an example in which a series of the processing are shared and executed by the control unit 10 of the information processing device 1 and the control unit 20 of the detection device 2 in the abovementioned processing has been described, however, the sharing of these by the control unit 10 and the control unit 20 is one example, and the abovementioned processing is not limited to the example of Fig. 6. In other words, the detection device 2 may substantially include the information processing device 1. Alternatively, the control unit 20 of the detection device 2 only outputs the detection value detected by the motion detection sensor 2a, and the control unit 10 of the information processing device 1 may perform the subsequent processing. Alternatively, the control unit 10 of the information processing device 1 and the control unit 20 of the detection device 2 perform inter-process communication, for example, and thus may execute a series of the processing in cooperation with each other.

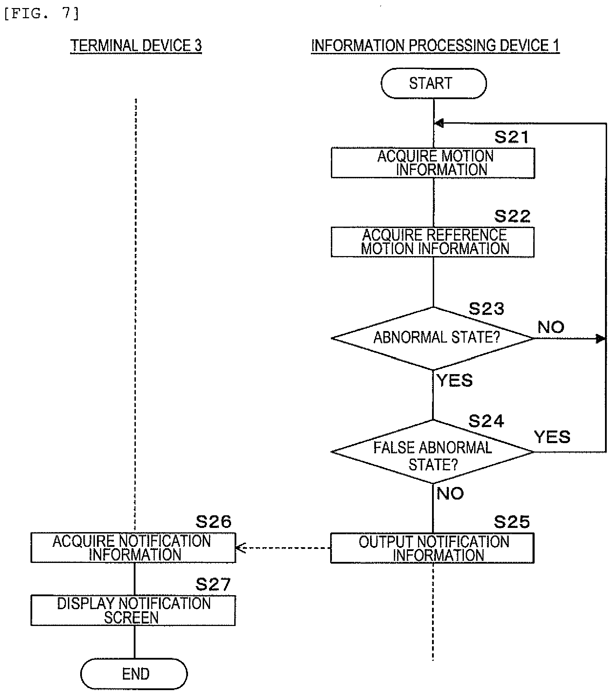

By using the reference motion information acquired as the above, the information processing device 1 determines whether there is a possibility of cerebral infarction from the motion information on the subject. Fig. 7 is a flowchart illustrating one example of a determination processing procedure of an abnormal state. The processing in Fig. 7 is executed by the control unit 10 of the information processing device 1 and the control unit 30 of the terminal device 3. The execution timing of the processing is, for example, timing at which new motion information is stored in the detection value DB 112.

The control unit 10 of the information processing device 1 refers to the detection value DB 112, and acquires new motion information in real time acquired and stored by the detection device 2 (Step S21). The motion information is associated with a subject ID, a motion pattern, and the like. The control unit 10 acquires reference motion information to be compared with the acquired motion information (Step S22). The control unit 10 refers to the reference motion information DB 113, and acquires reference motion information including a subject ID and a motion pattern identical to those in the acquired motion information.

The control unit 10 compares the acquired motion information with the reference motion information, and determines whether there is a possibility of cerebral infarction in a subject, in other words, whether the motion information on the subject is in an abnormal state (Step S23). If the control unit 10 detects, by analyzing left motion information in the left half body and right motion information in the right half body, a motion different from that in normal times in either one of the left half body and the right half body of the subject, the control unit 10 determines that an abnormal state where there is a possibility of cerebral infarction is present.

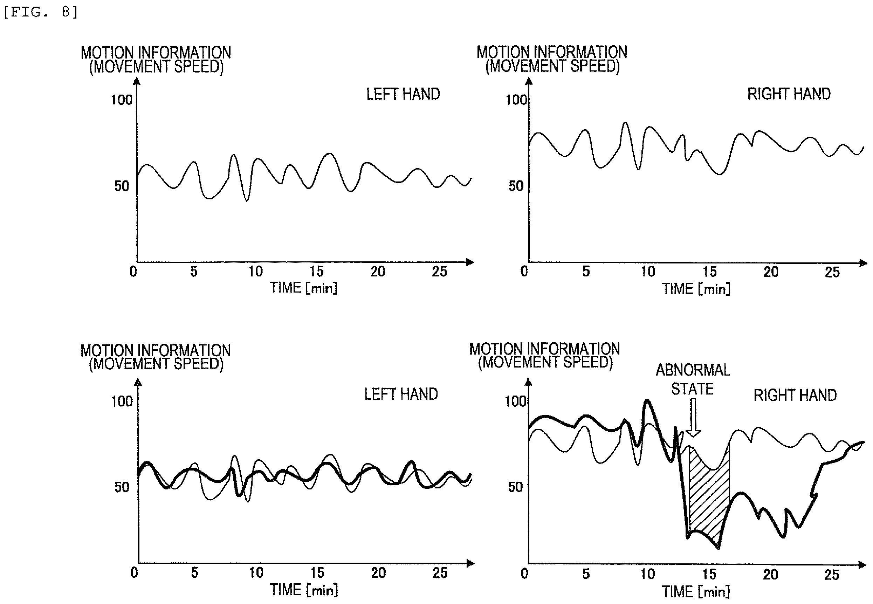

Specifically, the control unit 10 compares the acquired left motion information with left reference motion information in the reference motion information and compares the acquired right motion information with right reference motion information in the reference motion information, and determines whether a difference between the motion information and the reference motion information is equal to or more than the threshold value in each of the left half body and the right half body. Fig. 8 illustrates explanation diagrams related to determination processing of an abnormal state. Fig. 8 conceptually illustrates time-series changes in reference motion information (for example, the movement speed of the left hand and the right hand) in the left half body and the right half body on the upper side, and the time-series changes to which motion information on the left half body and the right half body at present is added on the lower side. In each graph in Fig. 8, the longitudinal axis indicates the motion information, and the horizontal axis indicates the time.

As mentioned above, the control unit 10 derives reference motion information on each of the left and right half bodies in a predetermined period from the collected motion information, and stores the reference motion information in the reference motion information DB 113. In a case where the control unit 10 has acquired motion information from the detection device 2, the control unit 10 compares motion information on each of the left and right half bodies with reference motion information, and calculates a difference value with the reference motion information in each of the left half body and the right half body. In other words, the control unit 10 calculates a difference between right reference motion information illustrated by solid line and current right motion information illustrated by thick line, in Fig. 8. Similarly, the control unit 10 calculates a difference between left reference motion information and current left motion information.

The control unit 10 compares the calculated difference value of each of the left and right half bodies with a predetermined threshold value, and determines whether an abnormality is present in the left half body and the right half body for each of the left half body and the right half body. Specifically, the control unit 10 determines that the abnormality is present if the difference value is equal to or more than the threshold value. Note that, for example, the control unit 10 may make a determination by comparing a difference of an instantaneous value at certain time with a threshold value, or may make a determination by comparing an integral value or a mean value of differences for a certain unit time (for example, five minutes) with a threshold value. The control unit 10 eventually determines whether the subject is in the abnormal state where there is a possibility of cerebral infarction based on a determination result of each of the left half body and the right half body. Specifically, if the control unit 10 has determined that an abnormality is present in one of the half bodies and no abnormality is present in the other half body, the control unit 10 determines that the abnormal state is present. If the control unit 10 has determined that no abnormality is present in both of the half bodies, the control unit 10 determines that no abnormal state is present. Moreover, also if the control unit 10 has determined that the abnormality is present in both of the half bodies, the control unit 10 determines that no abnormal state is present. For example, when a difference with the reference motion information is occurred as for both of the left and right motion information, it is determined that a possibility of cerebral infarction is low because the abnormality only in one side is not present.

Referring back to Fig. 7, the explanation is continuously made. If the control unit 10 has determined that the motion information is not in an abnormal state (Step S23: NO), the control unit 10 causes the processing return to Step S21, and continues the acquisition of motion information. If the control unit 10 has determined that the motion information is in an abnormal state (Step S23: YES), the control unit 10 determines whether the abnormal state is a false abnormal state (Step S24).

The false abnormal state herein is a state in which a predetermined change temporarily occurs in a motion of the left half body or the right half body due to a factor other than the cerebral infarction, and is a state having a low possibility of cerebral infarction, which is different from the abnormal state. Examples of factors of the false abnormal state can include, for example, numbness caused by the temporary and partial compression of the blood flow and the nerve due to sleeping for a long period of time with either one of the left half body or the right half body lying down, and muscle pain in only one side due to exercise and the like. Similar to the abnormal state, motion information having a left-right difference is detected from a subject in these false abnormal states in some cases.

The control unit 10 stores a false abnormal state information DB (not illustrated) that stores therein false abnormal state information in which motion information in a false abnormal state has been defined in advance. In the false abnormal state information DB, motion information for defining a false abnormal state to be indicated in a predetermined motion and other conditions are stored. The control unit 10 compares motion information in the false abnormal state information DB with the acquired motion information on the subject, and determines whether the abnormal state of the subject is a false abnormal state. For example, a case where the time when one side of the body lies down at sleeping is equal to or more than a predetermined value and a left-right difference of the difference value between left motion information or right motion information and reference motion information is less than a predetermined value, corresponds to false abnormal state information. Note that, the control unit 10 may determine whether the abnormal state is a false abnormal state by using the method of machine learning, in addition thereto. The control unit 10 may create and store in advance a learning model in which an identification result as to whether the abnormal state is a false abnormal state is output in a case where motion information is input, in the storage unit 11, and may determine whether the abnormal state is a false abnormal state from the learning model.

If the control unit 10 has determined that the abnormal state is a false abnormal state because the motion information on the subject corresponds to the false abnormal state information, in other words, a possibility of cerebral infarction is low (Step S24: YES), the control unit 10 causes the processing to return to Step S21, and continues the acquisition of motion information.

If the control unit 10 has determined that the abnormal state is not a false abnormal state because the motion information on the subject does not correspond to false abnormal state information, in other words, a possibility of cerebral infarction is high (Step S24: NO), the control unit 10 outputs notification information for making a notification about the occurrence of the abnormal state, to the terminal device 3, by the communication unit 12 (Step S25). The control unit 10 refers to the subject information DB 111 to specify a notification destination in a case where the subject is in an abnormal state, and outputs the notification information to the specified notification destination by using a mail function, for example.

The control unit 30 of the terminal device 3 acquires notification information for making a notification about the occurrence of the abnormal state, by the communication unit 32 (Step S26). The control unit 30 displays a notification screen 330 including the information indicating the occurrence of the abnormal state on the display unit 33, based on the acquired notification information (Step S27), and ends the series of the processing
Note that, the control unit 10 may perform loop processing in which the processing at Step S21 is executed after the processing at Step S25.

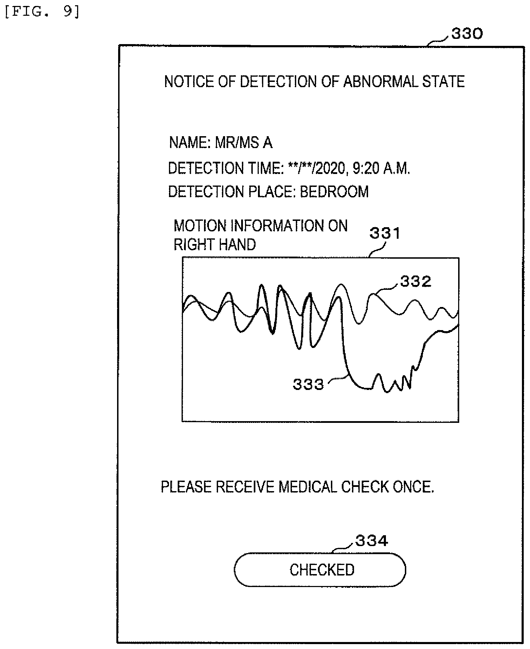

Fig. 9 is a view illustrating one example of the notification screen 330 that is displayed on the display unit 33. The control unit 30 displays notification screen information based on the notification information acquired from the information treatment device 1 on the display unit 33. The notification screen 330 includes the notification related to the motion information determined as being in an abnormal state. The notification screen 330 may further include a notification for prompting a check of the determination with respect to the motion information determined as being in an abnormal state. In the example illustrated in Fig. 9, the notification screen 330 includes information indicating detection date and time, a detection place, and the like of the motion information, and further include a motion graph 331 indicating the detected motion information, a check button 334 for prompting the check of the screen content, and the like. The notification screen 330 may further include a message for notifying the subject of support information (for example, the content for prompting the subject to have a consultation in a medical institution) in accordance with the determination result.

In the example illustrated in Fig. 9, on the notification screen 330, a time change of motion information (for example, the movement speed of the right hand) on a half body in which an abnormality is detected is illustrated in the motion graph 331 with the movement speed as the longitudinal axis and the time as the horizontal axis. The motion graph 331 includes a reference value curve 332 indicating reference motion information on the subject and a detection value curve 333 indicating motion information relating to the detected abnormal state. The control unit 10 of the information processing device 1 refers to reference motion information stored in the reference motion information DB 113 and motion information stored in the detection value DB 112, and generates and displays the reference value curve 332 and the detection value curve 333 in which time information is associated therewith, on the motion graph 331. Moreover, the control unit 10 refers to the detection value DB 112, acquires detection date and time, a detection place, and the like of the motion information relating to the abnormal state, generates text information in which the acquired detection date and time, detection place, and the like are displayed, and displays the text information on the notification screen 330. The control unit 10 may further read a detection value relating to the abnormal state detected by the motion detection sensor 2a from the detection value DB 112, and may display the read detection value with the motion graph 331 on the notification screen 330. In addition to motion information in the abnormal state, for example, the detection value acquired from the image data is displayed in association with the motion information, whereby it is possible to recognize the state of the actual motion of the subject in more details.

The subject can recognize the occurrence of the abnormal state on the notification screen 330 that is displayed in the display unit 33. This can make a notification of a possibility of cerebral infarction even in a case where the subject himself/herself is not aware, so that it is possible to prompt an early medical check and consultation, and can assist the detection of cerebral infarction. The subject checks the notification screen, and then clicks the check button 334. In a case where the control unit 30 of the terminal device 3 has received the pressing-down operation of the check button 334, the control unit 30 transmits operation information to the information processing device 1. The control unit 10 of the information processing device 1 acquires the operation information. In a case where the operation information is not acquired for a predetermined period, the control unit 30 may output again the notification information to the terminal device 3. This can reliably notify the subject of the information.

Note that, in the above, a difference value is determined for each of the left half body and the right half body, however, the present embodiment is not limited thereto. Specifically, the control unit 10 may determine whether the abnormal state is present based on a difference value between the left motion information and the right motion information.

In this case, the control unit 10 derives a difference value between left motion information and right motion information serving as reference motion information, by calculating, from the left motion information and the right motion information in a predetermined period, a difference value therebetween at each time, and obtaining the average of difference values for the period. The control unit 10 calculates a difference value between left motion information and right motion information in a case where the control unit 10 has acquired motion information from the detection device 2. The control unit 10 compares the calculated difference value with the difference value indicated by the reference motion information, and calculates a difference value (for convenience, called "second difference value") therebetween.

The control unit 10 compares the calculated second difference value with a threshold value, and determines that the abnormal state is present if the second difference value is equal to or more than the threshold value. In this manner, the control unit 10 may determine whether the abnormal state is present by comparing the left-right difference (difference value serving as the reference motion information) of the motion information in the reference state with the left-right difference in the current motion information.

Note that, the example in which the determination processing of an abnormal state and the determination processing of a false abnormal state are executed by the different processing has been described in the above, however, these determination processing may be executed as one processing. In other words, the processing at Step S23 may include the processing at Step S24, and the control unit 10 may determine whether an abnormal state is present by the determination processing of a false abnormal state. For example, the control unit 10 may determine that the subject has a low possibility of cerebral infarction, in other words, is not in an abnormal state by determining that the subject is in a false abnormal state based on the motion information.

Moreover, in the abovementioned determination of an abnormal state, in a case where several types of motion information are detected by a plurality of the motion detection sensors 2a, the information processing device 1 may determine whether an abnormal state is present for each of the several types of the motion information. For example, the information processing device 1 determines whether an abnormal state is present for each of motion information to be derived from image data by the camera and motion information to be derived from line-of-sight data by a line-of-sight sensor, so that it is possible to further improve the determination accuracy.

Moreover, as for the threshold value that is used for the abovementioned determination of an abnormal state, different threshold values may be defined in accordance with detection places of motion information, or the threshold value may be corrected in accordance with the detection place. For example, even for the identical motion pattern, the intensity, the movable range, and the like may change in accordance with the place where the motion is performed, so that it is preferable to use a threshold value in accordance with the detection place of the motion information and perform comparison with reference motion information. Moreover, for example, in a case where an ambient temperature in the surrounding of a subject is detected by using a temperature sensor or the like provided to the motion detection sensor 2a, a threshold value may be corrected based on a change in ambient temperature in a movement path of the subject. In a case where a temperature change equal to or more than a predetermined value has been detected in the surrounding of the subject, the motion content may change for the same motion pattern. The control unit 10 acquires a temperature change, performs comparison with reference motion information using a threshold value corrected by using a different correction value depending on the acquired temperature change, and determines the presence or absence of an abnormal state.

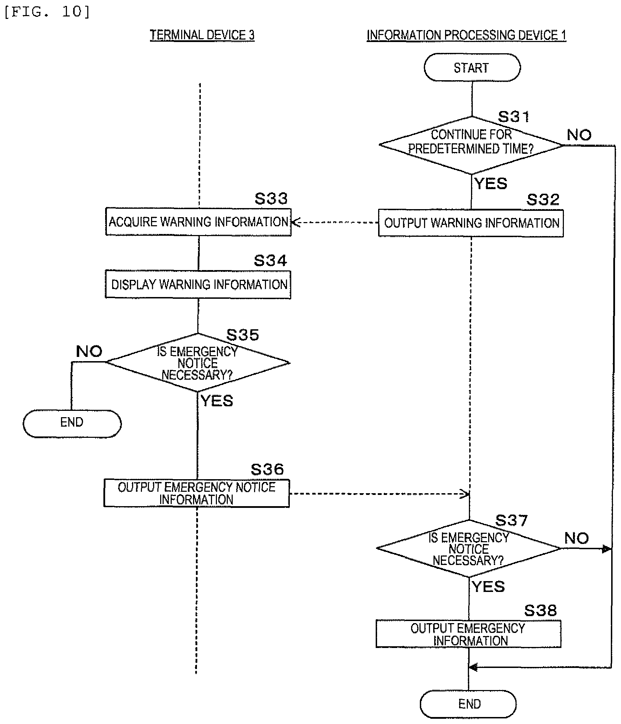

In a case where the abnormal state continues, the information processing device 1 outputs an additional notification. Fig. 10 is a flowchart illustrating one example of a processing procedure relating to warning information. The processing in Fig. 10 is executed by the control unit 10 of the information processing device 1 and the control unit 30 of the terminal device 3. If it has been determined that the subject is in the abnormal state at Step S24 in Fig. 7, the following processing is executed in parallel to the processing in Fig. 7.

The control unit 10 determines whether the abnormal state continues for a predetermined time (Step S31). The control unit 10 refers to the detection value DB 112, and specifies the time when first motion information relating to the abnormal state is detected. The control unit 10 determines a magnitude relationship between an elapsed time from the time when the first motion information is detected and a threshold value set in advance (for example, 20 minutes), and determines whether the elapsed time is equal to or more than the threshold value.

If the control unit 10 has determined that the abnormal state does not continue for the predetermined time because the elapsed time is less than the threshold value (Step S31: NO), the control unit 10 ends the processing. In this case, the control unit 10 may output information making a notification of the end of the abnormal state to the terminal device 3, by the communication unit 12.

If the control unit 10 has determined that the abnormal state continues for the predetermined time because the elapsed time is equal to or more than the threshold value (Step S31: YES), the control unit 10 may output warning information for making a notification of the continuation of the abnormal state to the terminal device 3, by the communication unit 12 (Step S32). The output destination of the warning information is the terminal device 3 of the subject, similar to the output destination of the notification information.

The control unit 30 of the terminal device 3 acquires warning information by the communication unit 32 (Step S33) . The control unit 30 displays, based on the acquired warning information, a warning screen 340 that receives the warning information and an emergency notice operation for the warning information, on the display unit 33 (Step S34).

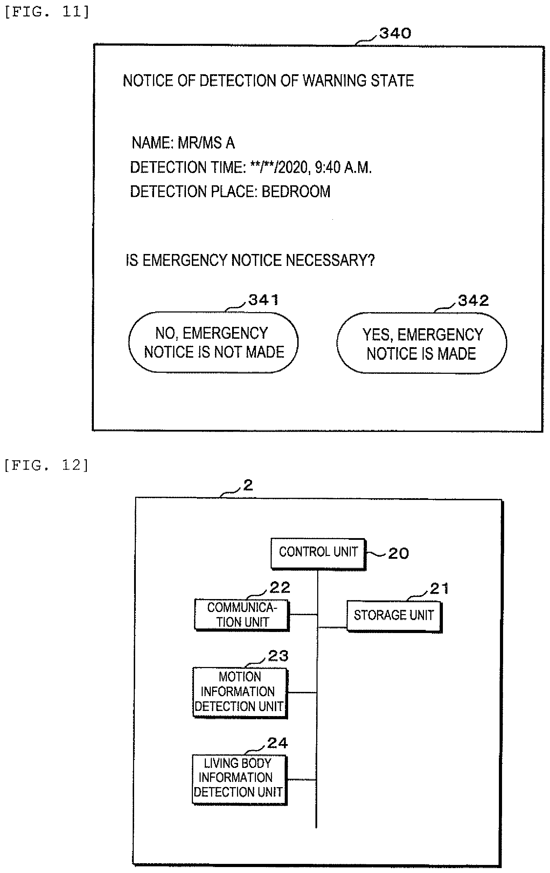

Fig. 11 is a view illustrating one example of the warning screen 340 that is displayed on the display unit 33. The control unit 30 displays warning screen information based on the warning information acquired from the information treatment device 1 on the display unit 33. The warning screen 340 includes warning information indicating the occurrence and the content of the warning state, and further includes a notification unnecessary button 341 for receiving the selection that an emergency notice for the warning information is unnecessary and a notification necessary button 342 for receiving the selection that an emergency notice for the warning information is necessary. The emergency notice indicates that in a case where the abnormal state of the subject continues, a high possibility of cerebral infarction is notified to others except the subject. The emergency notice assists a request for support to the subject in an emergency state, by the report to a family of the subject, a fire department, and a medical institution, for example. The subject performs a button operation of selecting the presence or absence of an emergency notice on the warning screen 340 that is displayed on the display unit 33. The control unit 30 receives the button operation, and executes processing in accordance with the received operation content.

Referring back to Fig. 10, the explanation is continuously made. The control unit 30 determines whether an emergency notice is necessary (Step S35) . If the control unit 30 has determined that the emergency notice is not necessary by receiving the selection operation of the notification unnecessary button 341 with the operation unit 34 (Step S35: NO), the control unit 30 ends the processing. On the other hand, if the control unit 30 has determined that the emergency notice is necessary by receiving the selection operation of the notification necessary button 342 with the operation unit 34 (Step S35: YES), the control unit 30 outputs emergency notice information indicating that the emergency notice is necessary to the information processing device 1, by the communication unit 32 (Step S36). The control unit 30 may determine that the emergency notice is necessary when an selection operation for neither of the notification unnecessary button 341 nor the notification necessary button 342 is received, and a predetermined time has elapsed.

The control unit 10 of the information processing device 1 determines whether an emergency notice for the warning information is necessary (Step S37). If the control unit 10 has determined that the emergency notice is necessary by having acquired the emergency notice information from the terminal device 3 (Step S37: YES), the control unit 10 outputs emergency information for making a notification of the abnormal state in the subject to the outside, by the communication unit 32 (Step S38). The control unit 10 may refer to the subject information DB 111 to specify an emergency notification destination in an emergency, and may make a notification to the emergency notification destination using a telephone call function, for example. If the control unit 10 has determined that the emergency notice is not necessary by having not acquired the emergency notice information from the terminal device 3 (Step S37: NO), the control unit 10 skips the emergency notice processing at Step S38, and ends the series of the processing.

In the abovementioned processing, the information processing device 1 preferably changes an acquisition interval of the motion information in accordance with a state of the subject. For example, at Step S23 in Fig. 7, if the control unit 10 has determined that the motion information is in an abnormal state, the control unit 10 outputs a change instruction of a detection interval to the detection device 2. For example, the acquisition interval of the motion information that is set as for every minute in normal times is changed to for every 10 seconds after the occurrence of the abnormal state, so that it is possible to identify a state of the subject in more details.

Note that, in the present embodiment, in the processing having described in the abovementioned respective flowcharts in Figs. 7 and 10, a part or all of the processing executed by the control unit 10 of the information processing device 1 may be executed by the control unit 30 of the terminal device 3, or may be executed by the control unit 20 of the detection device 2.

In the present embodiment, the terminal device 3 substantially may include the information processing device 1, and may perform communication directly with the detection device 2. Alternatively, the terminal device 3 may be configured to substantially include the information processing device 1 and the detection device 2, and one terminal device 3 having these functions may execute the abovementioned respective processing.

With the present embodiment, it is possible to determine a possibility of cerebral infarction based on a daily motion of the subject, specially motion information related to a motion to be repeatedly performed on a daily basis. When an abnormal state where there is a possibility of cerebral infarction has been detected, the subject is immediately notified, so that it is possible to assist the early detection of cerebral infarction. It is possible to more accurately determine whether an abnormal state is present by using the reference motion information and the false abnormal state information in accordance with individual subjects.

### (Second Embodiment)

In a second embodiment, living body information on a subject is acquired, and the presence or absence of a possibility of cerebral infarction in the subject is determined based on motion information and the living body information. Hereinafter, points in the second embodiment different from those in the first embodiment will be described. Other configurations except the configuration to be described later are similar to those of the information processing system 100 in the first embodiment, and the common configurations are assigned with the identical reference numerals and detailed explanations thereof are omitted.

The detection device 2 in the second embodiment is provided with the motion detection sensor 2a and the living body information detection sensor 2b, as illustrated in Fig. 1. The living body information detection sensor 2b is a detection device that detects living body information on a subject. The detection device 2 transmits motion information detected by the motion detection sensor 2a and living body information detected by the living body information detection sensor 2b, to the information processing device 1.

The living body information detection sensor 2b is, for example, an infrared ray camera that detects a blood flow and a surface temperature of the subject. The living body information detection sensor 2b may detect a body pressure of the subject by using a surface pressure sensor that is provided to a mat such as bedding and a seat that is used by the subject. In a case where the detection device 2 is a wearable device, the living body information detection sensor 2b may detect a pulse wave, a body temperature, and the like of the subject by using a pulse wave sensor, a temperature sensor, and the like. Note that, the detection device 2 may be configured to include a combination of the motion detection sensors 2a of several types and the living body information detection sensors 2b of several types.

Fig. 12 is a block diagram illustrating a configuration of the detection device 2 in the second embodiment. The detection device 2 is provided with a living body information detection unit 24, in addition to the control unit 20, the storage unit 21, the communication unit 22, and the motion information detection unit 23. The living body information detection unit 24 acquires a detection signal by using the living body information detection sensor 2b. The living body information detection unit 24 may have an A/D conversion function, and may output a measurement value obtained from the living body information detection sensor 2b, to the control unit 20.

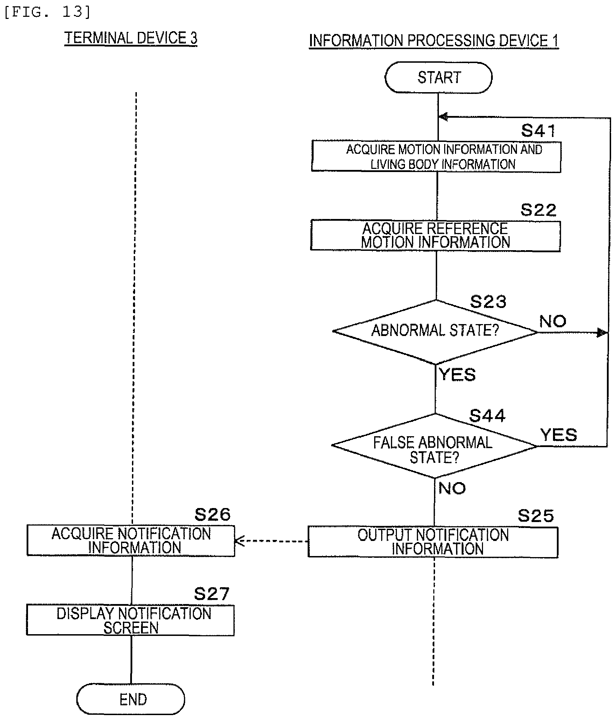

In the second embodiment, the control unit 10 of the information processing device 1 stores detection values including the motion information and the living body information acquired from the detection device 2, in the detection value DB 112. The information processing device 1 determines a possibility of cerebral infarction in the subject based on the motion information and the living body information. Fig. 13 is a flowchart illustrating one example of a determination processing procedure of an abnormal state in the second embodiment. The processing common to that of Fig. 7 in the first embodiment is assigned with the identical step number, and a detailed explanation thereof is omitted.

The control unit 10 of the information processing device 1 refers to the detection value DB 112, and acquires in real time new motion information and new living body information acquired and stored by the detection device 2 (Step S41). The motion information is associated with a subject ID, a motion pattern, and the like. The control unit 10 acquires reference motion information to be compared with the acquired motion information (Step S22). The control unit 10 refers to the reference motion information DB 113, and acquires reference motion information including a subject ID and a motion pattern identical to those in the acquired motion information.

The control unit 10 compares the acquired motion information with the reference motion information, and determines whether there is a possibility of cerebral infarction in a subject, in other words, whether the motion information on the subject is in an abnormal state (Step S23). If the control unit 10 has determined that the motion information is not in an abnormal state (Step S23: NO), the control unit 10 causes the processing return to Step S41, and continues the acquisition of motion information and living body information. If the control unit 10 has determined that the motion information is in an abnormal state (Step S23: YES), the control unit 10 determines whether the abnormal state is a false abnormal state (Step S44) .

The control unit 10 compares false abnormal state information related to a false abnormal state and stored in advance with the motion information on the subject, and determines whether the abnormal state in the subject is a false abnormal state. The control unit 10 in the second embodiment determines whether the abnormal state in the subject is a false abnormal state based on the motion information and the living body information. In other words, the false abnormal state information is defined by including motion information and living body information. For example, in a case where a change value of the blood flow amount, a body temperature, change values in shape of left and right half bodies, and the like of the subject are predetermined values, the motion information on the subject corresponds to the false abnormal state information.

If the control unit 10 has determined that the abnormal state is a false abnormal state because the motion information on the subject corresponds to the false abnormal state information (Step S44: YES), the control unit 10 causes the processing to return to Step S41 and continues the acquisition of motion information. If the control unit 10 has determined that the abnormal state is not a false abnormal state because the motion information on the subject does not correspond to the false abnormal state information (Step S44: NO), the control unit 10 outputs notification information for making a notification about the occurrence of the abnormal state, to the terminal device 3, by the communication unit 12 (Step S25).

The control unit 30 of the terminal device 3 acquires notification information for making a notification about the occurrence of the abnormal state, by the communication unit 32 (Step S26). The control unit 30 displays a notification screen including the information indicating the occurrence of the abnormal state on the display unit 33, based on the acquired notification information (Step S27), and ends the series of the processing. Note that, the control unit 10 may perform loop processing in which the processing at Step S21 is executed after the processing at Step S25.

With the present embodiment, for example, even in a case where it is difficult to determine whether the abnormal state is an abnormal state or a false abnormal state from only the motion information, a possibility of cerebral infarction is determined by including the motion information and the living body information, so that a suitable determination result can be provided.

### (Third Embodiment)

In a third embodiment, information processing is implemented by specializing motion information that is voice data. Hereinafter, points in the third embodiment different from those in the first embodiment will be described. Other configurations except the configuration to be described later are similar to those of the information processing system 100 in the first embodiment, and the common configurations are assigned with the identical reference numerals and detailed explanations thereof are omitted.

The detection device 2 in the third embodiment is provided with a sound sensor that detects speech sound of a subject, as the motion detection sensor 2a. The motion detection sensor 2a may be further provided with a camera or the like that detects a motion of a face of the subject.

The information processing device 1 determines a possibility of cerebral infarction based on motion information that is voice data acquired from the detection device 2. In the third embodiment, voice data relating to words of a plurality of patterns serving as the reference is stored in the reference motion information in the reference motion information DB 113. The information processing device 1 compares voice data on the subject in normal times with voice data detected in real time, and determines a possibility of cerebral infarction.

The control unit 10 executes an analysis of voice data detected by the sound sensor, and extracts a word pattern set in advance from the speech sound. The control unit 10 determines a magnitude relationship of a difference value between voice data corresponding to the extracted word pattern and voice data serving as reference motion information, and a threshold value set in advance, and determines, by determining whether the difference value is equal to or more than the threshold value, whether the abnormal state having a possibility of cerebral infarction is present.

The control unit 10 may further acquire image data indicating a speech motion of a face of the subject imaged by the camera. For example, by using a pattern matching method and other publicly known techniques, the control unit 10 performs an image analysis of the motion information that is the acquired image data, and determines whether the image data corresponds to the speech pattern set in advance. If the control unit 10 has determined that the image data corresponds to the speech pattern, the control unit 10 executes analysis processing of the abovementioned voice data, and determines whether the abnormal state is present.

With the present embodiment, for example, even in a case where the determination is difficult from a left-right difference of the motions in the body, a possibility of cerebral infarction can be determined based on the much sound information that occurs on a daily basis.

The scope of the present invention is indicated by the claims.

### Reference Signs List

1 information processing device
2 detection device
2a motion detection sensor
2b living body information detection sensor
3 terminal device
10, 20, 30 control unit
11, 21, 31 storage unit
111 subject DB
112 inspection value DB
113 reference motion information DB
1P program

## Claims

1. An information processing device (1), comprising:
a motion information acquisition unit that is configured to acquire motion information detected by a motion detection device (2a) that detects a motion of a subject;
a storage unit (21) that is configured to store therein the motion information acquired by the motion information acquisition unit;
a control unit (10);
wherein the control unit (10) is configured to derive reference motion information on a left half body and reference motion information on a right half body of the subject, based on the motion information in a predetermined period stored by the storage unit; and
the control unit (10) is configured to determine whether an abnormal state in which there is a possibility of cerebral infarction in the subject is present based on the reference motion information, and motion information on a left half body and motion information on a right half body of the subject at a detection time subsequent to the predetermined period
wherein the motion information includes first motion information in which a motion in the left half body of the subject is detected and second motion information in which a motion in the right half body of the subject is detected,
the control unit (10) is configured to respectively compare the first motion information in the reference motion information with the first motion information at the detection time, and the second motion information in the reference motion information with the second motion information at the detection time,
wherein, if the control unit (10) has determined that an abnormality is present in one of the half bodies and no abnormality is present in the other half body, the control unit (10) determines that the abnormal state is present, and if the control unit (10) has determined that no abnormality is present in both of the half bodies, the control unit (10) determines that no abnormal state is present, and also if the control unit (10) has determined that the abnormality is present in both of the half bodies, the control unit (10) determines that no abnormal state is present.

2. The information processing device (1) according to claim 1 that is configured to cause the control unit (10) to further execute processing of outputting a notification when it has been determined that the abnormal state is present.

3. The information processing device (1) according to claim 1 or 2 that is configured to cause the control unit (10) to further execute processing of determining whether the abnormal state continues for a predetermined time.

4. The information processing device (1) according to any one of claims 1 to 3 that is configured to cause the control unit (10) to further execute processing of determining, when it has been determined that the abnormal state is present, based on the motion information, whether a false abnormal state is present in which a predetermined change occurs in the left half body or the right half body of the subject due to a factor other than the cerebral infarction.

5. The information processing device (1) according to any one of claims 1 to 3 that is configured to cause the control unit (10) to further execute processing of:
acquiring living body information detected by a living body information detection device (2b) that detects the living body information on the subject; and
determining, when it has been determined that the abnormal state is present, based on the living body information, whether a false abnormal state is present in which a predetermined change occurs in the left half body or the right half body of the subject due to a factor other than the cerebral infarction.

6. The information processing device (1) according to any one of claims 1 to 5, wherein the motion information includes first motion information in which a motion in the left half body of the subject can be detected and second motion information in which a motion in the right half body of the subject can be detected, and the information processing device (1) is configured to cause the control unit (10) to further execute processing of determining, by comparing a difference value between the first motion information and the second motion information in the reference motion information with a difference value between the first motion information and the second motion information at the detection time, whether the abnormal state is present.

7. The information processing device (1) according to any one of claims 1 to 6 that is configured to cause the control unit (10) to further execute processing of comparing the reference motion information with the motion information at the detection time, using a threshold value that is different in accordance with a detection place of the motion information.

8. The information processing device (1) according to any one of claims 1 to 7, wherein the motion detection device (2a) includes several types of detection devices, and is configured to acquire a plurality pieces of motion information in the subject.

9. A computer program comprising instructions to cause the device of any one of claims 1 to 8 to execute processing of:
acquiring motion information detected by a motion detection device that detects a motion of a subject;
storing the acquired motion information in a storage unit;
deriving reference motion information on a left half body and reference motion information on a right half body of the subject, based on the stored motion information in a predetermined period; and
determining whether an abnormal state in which there is a possibility of cerebral infarction in the subject is present based on the derived reference motion information, and motion information on a left half body and motion information on a right half body of the subject at a detection time subsequent to the predetermined period,
wherein the motion information includes first motion information in which a motion in the left half body of the subject is detected and second motion information in which a motion in the right half body of the subject is detected,
respectively comparing the first motion information in the reference motion information with the first motion information at the detection time, and the second motion information in the reference motion information with the second motion information at the detection time,
if it has been determined that an abnormality is present in one of the half bodies and no abnormality is present in the other half body, it is determined that the abnormal state is present,
if it has been determined that no abnormality is present in both of the half bodies, it is determined that no abnormal state is present, and also
if it has been determined that the abnormality is present in both of the half bodies, it is determined that no abnormal state is present.

10. An information processing system (100), comprising: a motion detection device (2a) that detects a motion of a subject; and an information processing device (1) according to any one of claims 1 to 8 that acquires the motion information from the motion detection device (2a).

## Patentansprüche

1. Informationsverarbeitungsvorrichtung (1), umfassend:
eine Bewegungsinformationserfassungseinheit, die so konfiguriert ist, dass sie Bewegungsinformationen erfasst, welche von einer Bewegungserkennungsvorrichtung (2a) festgestellt werden, die eine Bewegung eines Subjekts detektiert;
eine Speichereinheit (21), die so konfiguriert ist, dass sie darin die Bewegungsinformationen speichert, die von der Bewegungsinformationserfassungseinheit erfasst wurden;
eine Steuerungseinheit (10);
wobei die Steuerungseinheit (10) so konfiguriert ist, dass sie Referenzbewegungsinformationen über eine linke Körperhälfte und Referenzbewegungsinformationen über eine rechte Körperhälfte des Subjekts auf der Grundlage der von der Speichereinheit gespeicherten Bewegungsinformationen in einer vorbestimmten Zeitspanne erlangt; und
die Steuerungseinheit (10) so konfiguriert ist, dass sie, auf der Grundlage der Referenzbewegungsinformationen und der Bewegungsinformationen über eine linke Körperhälfte und der Bewegungsinformationen über eine rechte Körperhälfte des Subjekts in einem Erkennungszeitraum, der auf die vorgegebene Zeitspanne folgt, festlegt, ob ein auffälliger Zustand vorhanden ist, in dem die Möglichkeit eines Hirninfarkts in dem Subjekt besteht,
wobei die Bewegungsinformationen erste Bewegungsinformationen, in denen eine Bewegung in der linken Körperhälfte des Subjekts festgestellt wird, und zweite Bewegungsinformationen, in denen eine Bewegung in der rechten Körperhälfte des Subjekts festgestellt wird, umfassen,
die Steuerungseinheit (10) so konfiguriert ist, dass sie jeweils die ersten Bewegungsinformationen in den Referenzbewegungsinformationen mit den ersten Bewegungsinformationen in dem Erkennungszeitraum und die zweiten Bewegungsinformationen in den Referenzbewegungsinformationen mit den zweiten Bewegungsinformationen in dem Erkennungszeitraum vergleicht, wobei, wenn die Steuerungseinheit (10) festgestellt hat, dass eine Auffälligkeit in einer der Körperhälften vorhanden ist und keine Auffälligkeit in der anderen Körperhälfte vorhanden ist, die Steuerungseinheit (10) festlegt, dass der auffällige Zustand vorhanden ist, und wenn die Steuerungseinheit (10) festgestellt hat, dass keine Auffälligkeiten in den beiden Körperhälften vorhanden sind, die Steuerungseinheit (10) festlegt, dass kein auffälliger Zustand vorhanden ist, und auch, wenn die Steuerungseinheit (10) festgestellt hat, dass die Auffälligkeit in den beiden Körperhälften vorhanden ist, die Steuerungseinheit (10) festlegt, dass kein auffälliger Zustand vorhanden ist.

2. Informationsverarbeitungsvorrichtung (1) nach Anspruch 1, die so konfiguriert ist, dass sie die Steuerungseinheit (10) veranlasst, ferner eine Verarbeitung zum Ausgeben einer Benachrichtigung durchzuführen, wenn festgestellt wurde, dass der auffällige Zustand vorhanden ist.

3. Informationsverarbeitungsvorrichtung (1) nach Anspruch 1 oder 2, die so konfiguriert ist, dass sie die Steuerungseinheit (10) veranlasst, ferner eine Verarbeitung zur Bestimmung durchzuführen, ob der auffällige Zustand für einen vorbestimmten Zeitraum andauert.

4. Informationsverarbeitungsvorrichtung (1) nach einem der Ansprüche 1 bis 3, die so konfiguriert ist, dass sie, wenn festgestellt wurde, dass der auffällige Zustand vorhanden ist, die Steuerungseinheit (10) veranlasst, ferner eine Verarbeitung durchzuführen, um auf der Grundlage der Bewegungsinformationen zu bestimmen, ob ein falscher auffälliger Zustand vorhanden ist, bei dem eine vorbestimmte Änderung in der linken Körperhälfte oder der rechten Körperhälfte des Subjekts aufgrund eines anderen Faktors als des Hirninfarkts auftritt.

5. Informationsverarbeitungsvorrichtung (1) nach einem der Ansprüche 1 bis 3, die so konfiguriert ist, dass sie die Steuerungseinheit (10) dazu veranlasst, ferner eine Verarbeitung durchzuführen von:
dem Erfassen von Informationen eines lebenden Körpers, die von einer Erkennungsvorrichtung (2b) für Informationen eines lebenden Körpers festgestellt werden, welche die Informationen eines lebenden Körpers auf dem Subjekt detektiert; und
dem Bestimmen, wenn bestimmt worden ist, dass der auffällige Zustand vorhanden ist, auf der Grundlage der Informationen über den lebenden Körper, ob ein falscher auffälliger Zustand vorhanden ist, bei dem eine vorbestimmte Änderung in der linken Körperhälfte oder der rechten Körperhälfte des Subjekts aufgrund eines anderen Faktors als dem Hirninfarkt auftritt.

6. Informationsverarbeitungsvorrichtung (1) nach einem der Ansprüche 1 bis 5, wobei die Bewegungsinformationen erste Bewegungsinformationen, in denen eine Bewegung in der linken Körperhälfte des Subjekts festgestellt werden kann, und zweite Bewegungsinformationen, in denen eine Bewegung in der rechten Körperhälfte des Subjekts festgestellt werden kann, umfassen, und die Informationsverarbeitungsvorrichtung (1) so konfiguriert ist, dass sie die Steuerungseinheit (10) veranlasst, ferner eine Verarbeitung durchzuführen, um durch das Vergleichen eines Differenzwertes zwischen den ersten Bewegungsinformationen und den zweiten Bewegungsinformationen in den Referenzbewegungsinformationen mit einem Differenzwert zwischen den ersten Bewegungsinformationen und den zweiten Bewegungsinformationen in dem Erkennungszeitraum zu bestimmen, ob der auffällige Zustand vorhanden ist.

7. Informationsverarbeitungsvorrichtung (1) nach einem der Ansprüche 1 bis 6, die so konfiguriert ist, dass sie die Steuerungseinheit (10) dazu veranlasst, ferner eine Verarbeitung zum Vergleichen der Referenzbewegungsinformationen mit den Bewegungsinformationen in dem Erkennungszeitraum unter Verwendung eines Schwellenwerts, der in Übereinstimmung mit einer Detektionsstelle der Bewegungsinformationen unterschiedlich ist, durchzuführen.

8. Informationsverarbeitungsvorrichtung (1) nach einem der Ansprüche 1 bis 7, wobei die Bewegungserkennungsvorrichtung (2a) mehrere Arten von Erkennungsvorrichtungen umfasst und so konfiguriert ist, dass sie eine Vielzahl von Bewegungsinformationen in dem Subjekt erfasst.

9. Computerprogramm, das Anweisungen umfasst, um die Vorrichtung nach einem der Ansprüche 1 bis 8 zu veranlassen, das Verarbeiten durchzuführen von:
dem Erfassen von Bewegungsinformationen, welche von einer Bewegungserkennungsvorrichtung festgestellt werden, die eine Bewegung eines Subjekts detektiert;
dem Speichern der erfassten Bewegungsinformationen in einer Speichereinheit;
dem Erlangen von Referenzbewegungsinformationen über eine linke Körperhälfte und von Referenzbewegungsinformationen über eine rechte Körperhälfte des Subjekts auf der Grundlage der gespeicherten Bewegungsinformationen in einer vorbestimmten Zeitspanne; und
dem Bestimmen, ob ein auffälliger Zustand vorliegt, in dem die Möglichkeit eines Hirninfarkts in dem Subjekt besteht, auf der Grundlage der erlangten Referenzbewegungsinformationen und der Bewegungsinformationen über eine linke Körperhälfte und der Bewegungsinformationen über eine rechte Körperhälfte des Subjekts in einem Erkennungszeitraum, der auf die vorbestimmte Zeitspanne folgt,
wobei die Bewegungsinformationen erste Bewegungsinformationen, in denen eine Bewegung in der linken Körperhälfte des Subjekts festgestellt wird, und zweite Bewegungsinformationen, in denen eine Bewegung in der rechten Körperhälfte des Subjekts festgestellt wird, umfassen,
dem entsprechenden Vergleichen der ersten Bewegungsinformationen in den Referenzbewegungsinformationen mit den ersten Bewegungsinformationen in dem Erkennungszeitraum und der zweiten Bewegungsinformationen in den Referenzbewegungsinformationen mit den zweiten Bewegungsinformationen in dem Erkennungszeitraum,
wenn festgestellt worden ist, dass in einer der Körperhälften eine Auffälligkeit vorhanden ist und in der anderen Körperhälfte keine Auffälligkeit vorhanden ist, wird festgelegt, dass der auffällige Zustand vorhanden ist,
wenn festgestellt worden ist, dass keine Auffälligkeiten in beiden Körperhälften vorhanden sind, wird festgelegt, dass kein auffälliger Zustand vorhanden ist, und auch
wenn festgestellt worden ist, dass in beiden Körperhälften die Auffälligkeit vorhanden ist, wird festgelegt, dass kein auffälliger Zustand vorhanden ist.

10. Informationsverarbeitungssystem (100), umfassend: eine Bewegungserkennungsvorrichtung (2a), die eine Bewegung eines Subjekts detektiert; und eine Informationsverarbeitungsvorrichtung (1) nach einem der Ansprüche 1 bis 8, welche die Bewegungsinformationen von der Bewegungserkennungsvorrichtung (2a) erfasst.

## Revendications

1. Dispositif de traitement d'informations (1), comprenant :
une unité d'acquisition d'informations de mouvement qui est configurée pour acquérir des informations de mouvement détectées par un dispositif de détection de mouvement (2a) qui détecte un mouvement d'un sujet ;
une unité de stockage (21) qui est configurée pour stocker, dans celle-ci, les informations de mouvement acquises par l'unité d'acquisition d'informations de mouvement ;
une unité de commande (10) ;
dans lequel l'unité de commande (10) est configurée pour déduire des informations de mouvement de référence sur une moitié gauche de corps et des informations de mouvement de référence sur une moitié droite de corps du sujet, sur la base des informations de mouvement dans une période prédéterminée stockées par l'unité de stockage ; et
l'unité de commande (10) est configurée pour déterminer si un état anormal dans lequel il existe une possibilité d'infarctus cérébral chez le sujet est ou non présent sur la base des informations de mouvement de référence, et des informations de mouvement sur une moitié gauche de corps et des informations de mouvement sur une moitié droite de corps du sujet à un temps de détection après la période prédéterminée,
dans lequel les informations de mouvement comportent des premières informations de mouvement dans lesquelles un mouvement dans la moitié gauche de corps du sujet est détecté et des deuxièmes informations de mouvement dans lesquelles un mouvement dans la moitié droite de corps du sujet est détecté,
l'unité de commande (10) est configurée pour comparer respectivement les premières informations de mouvement dans les informations de mouvement de référence aux premières informations de mouvement au temps de détection, et les deuxièmes informations de mouvement dans les informations de mouvement de référence aux deuxièmes informations de mouvement au temps de détection,
dans lequel, si l'unité de commande (10) a déterminé qu'une anomalie est présente dans l'une des moitiés de corps et aucune anomalie n'est présente dans l'autre moitié de corps, l'unité de commande (10) détermine que l'état anormal est présent et, si l'unité de commande (10) a déterminé qu'aucune anomalie n'est présente dans les deux moitiés de corps, l'unité de commande (10) détermine qu'aucun état anormal n'est présent et également, si l'unité de commande (10) a déterminé que l'anomalie est présente dans les deux moitiés de corps, l'unité de commande (10) détermine qu'aucun état anormal n'est présent.

2. Dispositif de traitement d'informations (1) selon la revendication 1, qui est configuré pour amener l'unité de commande (10) à exécuter en outre le traitement de délivrance d'une notification lorsqu'il a été déterminé que l'état anormal est présent.

3. Dispositif de traitement d'informations (1) selon la revendication 1 ou 2, qui est configuré pour amener l'unité de commande (10) à exécuter en outre le traitement de la détermination si l'état anormal continue ou non pendant un temps prédéterminé.

4. Dispositif de traitement d'informations (1) selon l'une quelconque des revendications 1 à 3, qui est configuré pour amener l'unité de commande (10) à exécuter en outre le traitement de la détermination, lorsqu'il a été déterminé que l'état anormal est présent, sur la base des informations de mouvement, si un faux état anormal est ou non présent dans lequel un changement prédéterminé survient dans la moitié gauche de corps ou dans la moitié droite de corps du sujet sous l'effet d'un facteur autre que l'infarctus cérébral.

5. Dispositif de traitement d'informations (1) selon l'une quelconque des revendications 1 à 3, qui est configuré pour amener l'unité de commande (10) à exécuter en outre le traitement de :
l'acquisition d'informations de corps vivant détectées par un dispositif de détection d'informations de corps vivant (2b) qui détecte les informations de corps vivant sur le sujet ; et
la détermination, lorsqu'il a été déterminé que l'état anormal est présent, sur la base des informations de corps vivant, si un faux état anormal est ou non présent dans lequel un changement prédéterminé survient dans la moitié gauche de corps ou dans la moitié droite de corps du sujet sous l'effet d'un facteur autre que l'infarctus cérébral.

6. Dispositif de traitement d'informations (1) selon l'une quelconque des revendications 1 à 5, dans lequel les informations de mouvement comportent des premières informations de mouvement dans lesquelles un mouvement dans la moitié gauche de corps du sujet peut être détecté et des deuxièmes informations de mouvement dans lesquelles un mouvement dans la moitié droite de corps peut être détecté, et le dispositif de traitement d'informations (1) est configuré pour amener l'unité de commande (10) à exécuter en outre le traitement de la détermination, par la comparaison d'une valeur de différence entre les premières informations de mouvement et les deuxièmes informations de mouvement dans les informations de mouvement de référence à une valeur de différence entre les premières informations de mouvement et les deuxièmes informations de mouvement au temps de détection, si l'état anormal est ou non présent.

7. Dispositif de traitement d'informations (1) selon l'une quelconque des revendications 1 à 6, qui est configuré pour amener l'unité de commande (10) à exécuter en outre le traitement de la comparaison des informations de mouvement de référence aux informations de mouvement au temps de détection, à l'aide d'une valeur de seuil qui est différente en fonction d'un lieu de détection des informations de mouvement.

8. Dispositif de traitement d'informations (1) selon l'une quelconque des revendications 1 à 7, dans lequel le dispositif de détection de mouvement (2a) comporte plusieurs types de dispositifs de détection, et est configuré pour acquérir une pluralité d'éléments d'informations de mouvement dans le sujet.

9. Programme informatique comprenant des instructions pour amener le dispositif selon l'une quelconque des revendications 1 à 8 à exécuter le traitement de :
l'acquisition d'informations de mouvement détectées par un dispositif de détection de mouvement qui détecte un mouvement d'un sujet ;
le stockage des informations de mouvement acquises dans une unité de stockage ;
la déduction d'informations de mouvement de référence sur une moitié gauche de corps et d'informations de mouvement de référence sur une moitié droite de corps du sujet, sur la base des informations de mouvement stockées dans une période prédéterminée ; et
la détermination si un état anormal dans lequel il existe une possibilité d'infarctus cérébral chez le sujet est ou non présent sur la base des informations de mouvement de référence déduites, et des informations de mouvement sur une moitié gauche de corps et des informations de mouvement sur une moitié droite de corps du sujet à un temps de détection après la période prédéterminée,
dans lequel les informations de mouvement comportent des premières informations de mouvement dans lesquelles un mouvement dans la moitié gauche de corps du sujet est détecté et des deuxièmes informations de mouvement dans lesquelles un mouvement dans la moitié droite de corps du sujet est détecté,
la comparaison respectivement des premières informations de mouvement dans les informations de mouvement de référence aux premières informations de mouvement au temps de détection, et des deuxièmes informations de mouvement dans les informations de mouvement de référence aux deuxièmes informations de mouvement au temps de détection, s'il a été déterminé qu'une anomalie est présente dans l'une des moitiés de corps et aucune anomalie n'est présente dans l'autre moitié de corps, il est déterminé que l'état anormal est présent,
s'il a été déterminé qu'aucune anomalie n'est présente dans les deux moitiés de corps, il est déterminé qu'aucun état anormal n'est présent, et également
s'il a été déterminé que l'anomalie est présente dans les deux moitiés de corps, il est déterminé qu'aucun état anormal n'est présent.

10. Système de traitement d'informations (100), comprenant : un dispositif de détection de mouvement (2a) qui détecte un mouvement d'un sujet ; et un dispositif de traitement d'informations (1) selon l'une quelconque des revendications 1 à 8 qui acquiert les informations de mouvement à partir du dispositif de détection de mouvement (2a) .
